# EUROPEAN PATENT APPLICATION

(11) **EP 1 710 346 A2**
(43) Date of publication of application: **11.10.2006**
(21) Application number: 06251646.3
(22) Date of filing: 28.03.2006
(51) Int. Cl.: D06M 15/53, A61L 15/26, D04H 3/16

(54) **Non-linting water-absorbent polypropylene spunbonded material, and method of forming same**

(30) Priority: 05.04.2005 US 99004
(71) Applicant: Promedical Products Co., Ltd., Changzhon, Jiangsu (CN)
(72) Inventor: Wu, Willie, Edmonton, Alberta T6R 2C7 (CA)
(74) Representative: Bibby, William Mark

(57) **Abstract**

The method of producing a non-linting water-absorbent polypropylene spun bound material suitable for use in medical and surgical applications and the like, comprising the steps of: forming a porous sheet of said spun bound material; and coating the individual fibers of said material with a hydrophillic compound; thereby to form a sheet of non-linting water-absorbent spun bound material suitable for use in medical and surgical applications and the like.

## Description

### Technical Field

The present invention relates to generally to the field of spun bound sheet materials suitable for use in medical and surgical applications and the like, and, more particularly, to improved non-linting water-absorbent polypropylene spun bound ("PSB") materials, and to improved methods of forming the same.

### Background Art

Poly spun bound materials are known. Because of the cost of manufacture is relatively low, such poly spun bound materials have a wide variety of possible applications. Because polypropylene is an inherently hydrophobic material, such PSB material is particularly suited for use in surgical drapes and garments.

It would be generally desirable to provide poly spun bound materials that are hydrophillic. Rather than resisting absorption, these hydrophillic PSB materials would absorb water. However, when used in medical and surgical applications and the like, such materials should be non-linting to prevent contamination of the surgical site.

### Disclosure of the Invention

With reference to the corresponding parts, portions or surfaces of the disclosed embodiment, merely for purposes of illustration and not by way of limitation, the present invention broadly provides an improved non-linting water-absorbent polypropylene spun bound material suitable for use in medical and surgical applications and the like, and to an improved method of forming same.

In one aspect, the invention provides an improved method of producing a non-linting water-absorbent polypropylene spun bound material suitable for use in medical and surgical applications and the like, which comprises the steps of: forming a porous sheet of polypropylene spun bound material, and coating the individual fibers of such material with a hydrophillic compound, thereby to form a sheet of non-linting water-absorbent spun bound material suitable for use in medical and surgical applications and the like.

In the preferred method, the porous sheet is formed of copolymer (as opposed to homopolymer) polypropylene. This material, prior to being coated, is inherently hydrophobic. The coating compound may be R-O-(CH₂CH₂O)_{nH}. The fibers of the porous sheet may be coated by immersion in a liquid solution of the hydrophillic compound, and then dried.

In another aspect, the invention provides a non-linting water-absorbent polypropylene spun bound material suitable for use in medical and surgical applications and the like, which comprises: a porous sheet of such spun bound material, and a hydrophillic compound coating the individual fibers of the material, whereby the coated sheet will be non-linting and water-absorbent and will be suitable for use in medical and surgical applications and the like.

The porous sheet may be formed of copolymer polypropylene, which, prior to being coated, is inherently hydrophobic. The coating compound may be R-O-(CH₂CH₂O)_{nH}.

Accordingly, the general obj ect of the invention is to provide an improved water-absorbent polypropylene spun bound material suitable for use in medical and surgical applications and the like.

Another object is to provide such a poly spun bound material that is non-linting.

Still another object is to provide an improved method of producing a non-linting water-absorbent polypropylene spun bound material.

These and other objects and advantages will be become apparent from the foregoing and ongoing written specification, the drawings and the appended claims.

### Brief Description of the Drawings

Fig. 1 is a schematic view of the apparatus used to form the improved water-absorbent poly spun bound material.
Fig. 2 is a schematic of the apparatus used to convert the PSB material form its hydrophobic to its hydrophillic form.
Fig. 3. is a flow chart showing the various steps involved in practice of the process showing in Fig. 1.
Fig. 4 is a flow chart showing the various steps involved in the conversion operation shown in Fig. 2.

### Description of the Preferred Embodiments

At the outset, it should be clearly understood that like reference numerals are intended to identify the same structural elements, portions or surfaces consistently throughout the several drawing figures, as such elements, portions or surfaces may be further described or explained by the entire written specification, of which this detailed description is an integral part. Unless otherwise indicated, the drawings are intended to be read (e.g., cross-hatching, arrangement ofparts, proportion, degree, etc.) together with the specification, and are to be considered a portion of the entire written description of this invention. As used in the following description, the terms "horizontal", "vertical", "left", "right", "up" and "down", as well as adjectival and adverbial derivatives thereof (e.g., "horizontally", "rightwardly", "upwardly", etc.), simply refer to the orientation of the illustrated structure as the particular drawing figure faces the reader. Similarly, the terms "inwardly" and "outwardly" generally refer to the orientation of a surface relative to its axis of elongation, or axis of rotation, as appropriate.

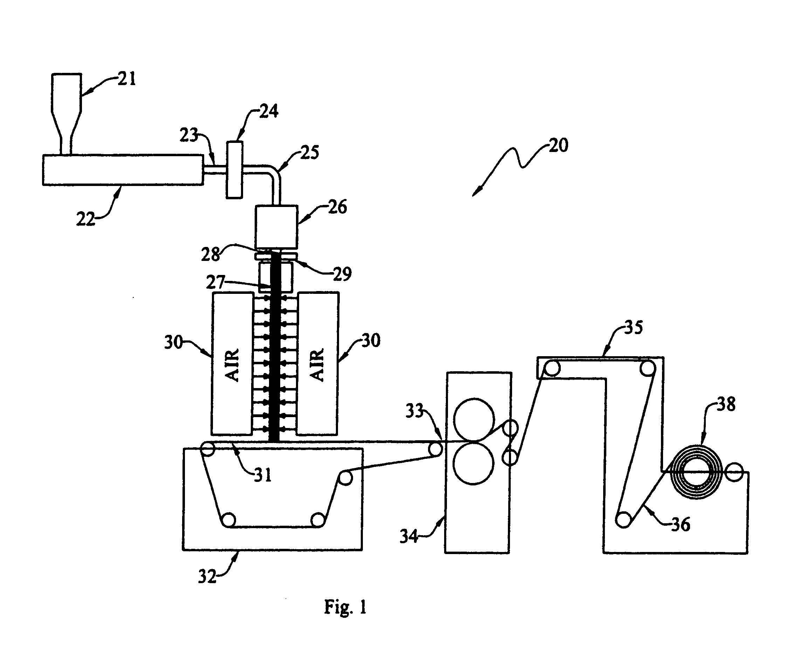

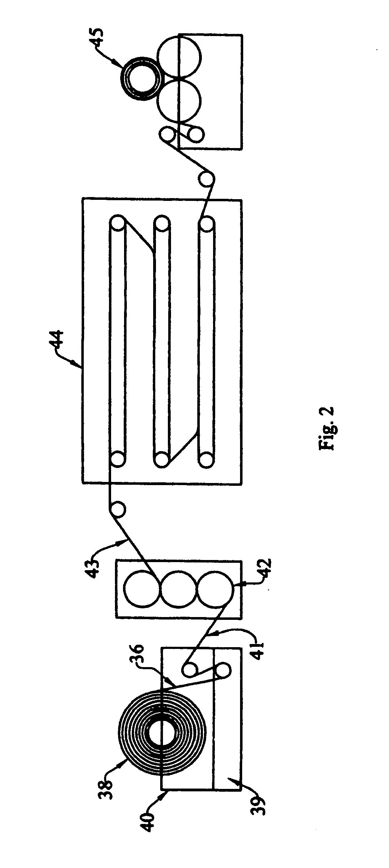

Referring to the drawings, the present invention broadly provides an improved method of producing a non-linting water-absorbent polypropylene spun bound material suitable for use in medical and surgical applications and the like. In another aspect, the invention provides an improved material produced in accordance with the foregoing process. Fig. 1 is a schematic of the apparatus used to produce PSB, and Fig. 2 is a schematic of the apparatus used to convert PSB material, which is inherently hydrophobic, to a modified form that is hydrophillic.

In Fig. 1, granular or pellet-like copolymer polypropylene material is supplied to, and deposited in, hopper 21. The granular material falls by gravity in the hopper and enters the left marginal end of a screw conveyor 22. This screw conveyor has a helical screw (not shown) arranged within a cylinder. In the well known manner, the adjacent convolutions of the screw are more closely spaced as material is conveyed from its left end to its right end. This causes frictional heating of the pellets. The helical screw has a nominal diameter of about 150 millimeters, and has a length-to-diameter ratio on the order of about 28:1 to 30:1. The screw is rotated in the range of about 20-80 rpm. The cylinder may have the supplemental external heating, if desired. When the screw rotates, the granular material is frictionally heated to a temperature of about 250 °C, causing the granular pellets to progressively rise in temperature and melt as they propagate along the cylinder. By the time it reaches the right end or exit end of screw conveyor 22, the supplied or input material, has been melted and liquified to a viscous liquid, and is at about 250 °C. Thereafter, the material is passes via conduit 23 to a filter 24. This filter may simply be a sieve- or mesh-like device that is used to remove dust and dirt from the liquid polypropylene.

From filter 24, the viscous polypropylene liquid passes through a conduit 25 to a pressure relief device 26. This device simply stabilizes the pressure of the liquid resin at about 60 bars.

Thereafter, the liquid resin passes via conduit 28 to an ejection head 29, where a plurality of long continuous filament strands, of approximately 40 microns diameter, are discharged downwardly. The ejection pressure is about 20 bars. The temperature of the ejected filaments is still about 250 °C. Thereafter, opposing air flows (represented by boxes 30, 30) are directed at the extruded filaments. The function of this air flow is to rapidly cool the filaments from their initial ejection temperature of about 250 °C (at their upper end) to about 145 °C (at their lower end). In other words, the extrudate is rapidly cooled as it moves downwardly for a length of about 1 meter. The cooling air flow is approximately 40 m' per minute. The nominal air pressure is 2 bars, and, in the preferred embodiment, it is substantially at room temperature (*i*.*e*., at about 22°C). As previously indicated, this mass flow of air quickly cools the extruded filaments 27 by about 105°C as they travel downwardly.

At their lower end, the extruded fibers then fall onto continuous stainless steel conveyor 31, traveling at between about 10 meters per minute to about 100 meters per minute. If desired, the pressure below the belt (represented by box 32) may be about -3 bars. In other words, there is a pressure differential of about 3 bars across the web, which causes the web to adhere closely to the conveyor. Thereafter, the moving conveyor delivers the sheet material 33 to a calendaring device 34. The sheet material passes between two opposed rolls, the sheet material still being at a temperature of about 145 °C. In the preferred embodiment, these rolls exert a pressure of about 50 newtons per square meter on the web so as to imprint a surface thereto and to give it a uniform appearance. Thereafter, after passing around certain other rolls (indicated at 35) to facilitate cooling, the calendared sheet material 36 is then wound on a roll, indicated at 38.

Polypropylene is a material that is inherently hydrophobic. This means that it does not absorb water. Rather, to the contrary, water tends to bead on this material.

Referring now to Fig. 2, to make the inherently-hydrophobic material 36 capable of absorbing water, such material is unwound form roll 38 and immersed in a liquid solution 39 of a hydrophillic compound. Since the poly spun bound material is porous, the liquid solution of the hydrophillic compound readily penetrates' and coats the individual fibers thereof. The amount of chemical added is up to about 0.5% by weight. After passing through the immersion bath 40, the wetted material, indicated at 41, is then passed through a serious of rolls, indicated at 42, that may be adjusted to selectively squeeze excess liquid therefrom. The roll pressure may be up to about 40 newtons/m². After passing through these rolls, the material, now indicated at 43, is then sent to a dryer 44. The drying oven may be a temperature of about 110 - 120 °C, and the transit time through the oven may be about 2-3 min. After passing through the dryer, the individual fibers are coated with the hydrophillic compound. The material is then rewound or a roll 45.

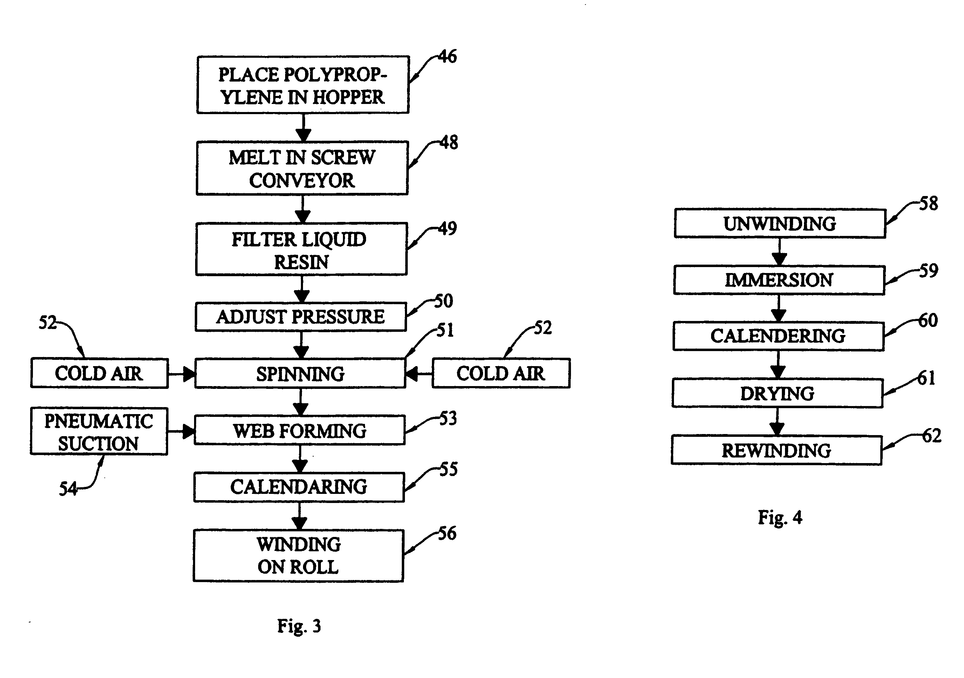

Fig. 3 is a flow chart showing the sequence of steps involved in the process. First, the granular or pellet-like polypropylene material is first placed into the hopper, indicated in box 46. Thereafter, the material is melted as it passes through the screw conveyor to form a liquid resin, as indicated in box 48. The liquid resin is then filtered, as indicated in box 49, and the pressure is stabilized, as indicated in box 50, via controlled by a pressure release device. Thereafter, the individual fibers are spun, as indicated in box 51, while being subjected to a cold air (i.e, at room temperature air flow), as indicated in boxes 52, 52, to form a web 53 on the surface of the moving conveyor. Suction may be applied to cause the web to more closely adhere to the conveyor, as indicated in box 54. Thereafter, the web is passed through the calendaring rolls, as indicated in box 55, and wound on a roll, as indicated in box 56.

Referring now to Fig. 4, to change the PSB material form one that is inherently hydrophobic to one that is hydrophillic, a roll of such material is then unwound, as indicated at 58, and passed through an immersion bath of a liquid solution of the hydrophillic compound, as indicated in box 59. The wetted web is then passed though calendaring rolls (as indicated at 60), dried (as indicated in box 61), then rewound on another roll (as indicated in box 62).

Thus, the present invention broadly provides and improved method of producing a non-linting water-absorbent polypropylene spun bound material suitable for use in medical and surgical applications and the like, which includes the steps of forming a porous sheet of spun bound material, and coating the individual fibers of such material with a hydrophillic compound, thereby to form a sheet of non-linting water-absorbent spun bound material suitable for use in medical and surgical applications and the like.

### Modifications

The present invention expressly contemplates that many changes and modifications may be made. For example, the input material may be either a pelletized or granular polypropylene material, however, the input material may take other forms as well. In the preferred embodiment, the input material is heated by a screw conveyor, either with or without the provision of an external heat source. However, other types of conveyors and heaters might be used as well. The liquified resin is then passed through a filter. In the preferred form, the filter is fine-mesh or sieve-like screen. However, other filters might be used. After having been filtered, the pressure is then stabilized, and a plurality of long continuous fibers are extruded onto a moving conveyor. The size, number, spacing and diameter of these fibers may be changed. The fibers may be cooled by a cold air bath, or by some other means as well. The fibers are preferably passed through a calendaring roll to imprint a pattern on the surface of the thus-formed web. However, different pressures might be used and/or different patterns might possibly be imprinted on the web. Thereafter, the moving web is cooled, and is wound on a roll. Other types of cooling other than by room temperature might also be used.

In the process of making the poly spun bound web hydrophillic, the roll is preferably immersed in a liquid solution of the hydrophillic compound. This is not invariable. In other embodiments, the coating material might be sprayed on to the moving web. The step of squeezing the wetted web between a serious of dies is simply to try to remove excess material and to improve the consistency and uniformity of the web. While the preferred embodiment contemplates that the wetted web be dried in an oven, this too is not invariable. The web could simply dried at room temperature, if desired. Thereafter, the product is rewound onto another roll.

Therefore, while the present invention provides an improved method of producing a non-linting water-absorbent polypropylene spun bound material, and to the material itself, certain modifications and changes may be made without departing from the spirit of the invention, as defined and differentiated by the following claims.

## Claims

1. The method of producing a non-linting water-absorbent polypropylene spun bound material, comprising the steps of:
forming a porous sheet of said spun bound material; and
coating the individual fibers of said material with a hydrophillic compound;
thereby to form a sheet of non-linting water-absorbent spun bound material suitable for use in medical and surgical applications.

2. The method as set forth in claim 1 wherein said porous sheet is formed of copolymer polypropylene.

3. The method as set forth in claim 1 wherein said porous sheet, prior to being coated, is inherently hydrophobic.

4. The method as set forth in claim 1 wherein said coating compound is R-O-(CH₂CH₂O)_{nH}.

5. The method as set forth in claim 1 wherein said porous sheet is coated by immersion in a liquid solution of said hydrophillic compound.

6. The method as set forth in claim 1 wherein said porous sheet is dried after immersion in said liquid solution of hydrophillic compound.

7. A non-linting water-absorbent polypropylene spun bound material, comprising:
a porous sheet of said spun bound material; and
a hydrophillic compound coating the individual fibers of said material;
whereby said coated sheet will be non-linting and water-absorbent and will be suitable for use in medical and surgical applications.

8. The material as set forth in claim 7 wherein said porous sheet is formed of copolymer polypropylene.

9. The material as set forth in claim 7 wherein said porous sheet, prior to being coated, is inherently hydrophobic.

10. The material as set forth in claim 1 wherein said coating compound is R-O-(CH₂CH₂O)_{nH}.
